(19) Europäisches Patentamt
European Patent Office
Office européen des brevets

(11) Veröffentlichungsnummer: **0 446 473 A2**

(12) # EUROPÄISCHE PATENTANMELDUNG

(21) Anmeldenummer: **90125012.6**

(22) Anmeldetag: **20.12.90**

(51) Int. Cl.$^5$: **A61L 25/00**, A61L 15/32, //C08L89:06

(30) Priorität: **10.03.90 DE 4007668**

(43) Veröffentlichungstag der Anmeldung: **18.09.91 Patentblatt 91/38**

(84) Benannte Vertragsstaaten: **AT BE DE ES FR GB IT NL SE**

(71) Anmelder: **Beiersdorf Aktiengesellschaft
Unnastrasse 48
W-2000 Hamburg 20(DE)**

(72) Erfinder: **Sachau, Günter
Lessingstrasse 21
W-1085 Quickborn(DE)**
Erfinder: **Borgschulte, Katrin, Dr.
Eppendorfer Weg 168
W-2000 Hamburg 20(DE)**
Erfinder: **Pietsch, Hanns, Dr.
Mittelweg 25
W-2000 Hamburg 13(DE)**

(54) **Hydrogelschäume und Verfahren zu deren Herstellung.**

(57) Hydrogelschäume,
a) basierend auf Gelatine und Wasser,
b) enthaltend gegebenenfalls Polyvinylalkohol
c) enthaltend einen Vernetzer, gewählt aus der Gruppe
ca) der Verbindungen mindestens dreiwertiger Metalle oder Halbmetalle, z.B. Borsäure, Borate, Aluminiumsalze, Titanate, Alkyltitanate und/oder der Gruppe der
cb) organischen und/oder anorganischen Säuren und/oder deren Salzen, z.B. Gallussäure, Gallate, Phosphorsäuren, Phosphate, Dihydroxybenzoesäure, Dihydroxybenzoate
d) sowie gegebenenfalls enthaltend organische Weichmacher, und/oder Hilfs- und/oder Zusatzstoffe.

EP 0 446 473 A2

Die vorliegende Erfindung betrifft Hydrogelschäume, insbesondere solche für medizinische Anwendungen, sowie Verfahren für ihre Herstellung.

Hydrogele sind makromolekulare, natürliche oder synthetische Stoffe die aufgrund eines hohen Gehaltes an hydrophilen Gruppen in der Lage sind, Wasser absorptiv zu binden. Die Wasseraufnahmekapazität vieler Hydrogele beträgt das Mehrfache des Eigengewichtes der wasserfreien Substanz.

Hydrogele werden in vielfältiger Form in der Medizin eingesetzt. Besonders geeignet sind sie in der Wundversorgung; sie können

* Wunden vor der Austrocknung schützen
* Wundsekret aufsaugen
* als Matrix für Wirkstoffe aller Art dienen
* als Basis für die Besiedelung mit autologen

oder heterologen Hautzellen dienen Hydrogele können unter anderem in Form von Schäumen verwendet werden.

Schäume zur Versorgung von Hautwunden oder chirurgischen Wunden sind an sich bekannt. Hauptsächlich finden dabei Polyurethanschäume oder Kollagenschäume Verwendung.

Die Hydrogele des Standes der Technik haben jedoch verschiedene Nachteile:
Aufgrund ihrer Hydrophilie sind die meisten in Frage kommenden Stoffe wasserlöslich. Dies ist meist unerwünscht, weil derartige Produkte nicht formstabil sind. Außerdem lösen sich derartige Produkte in unerwünschter Weise am Einsatzort auf und stehen dann für den vorgesehenen Zweck nicht mehr zur Verfügung.

Andere Produkte zeichnen sich durch starke Polymervernetzung aus. Dadurch werden zwar manche der Nachteile der vorgenannten Stoffklasse vermieden. Die Quellbarkeit dieser Stoffe ist jedoch weitgehend eingeschränkt oder verloren. Überdies sind die synthetischen Vernetzer, die hier eingesetzt werden, alle mehr oder weniger toxisch.

In EP-A-0 097 846 werden Wundbehandlungsmittel auf der Basis von Hydrogelen beschrieben. Dabei wird Gelatine in fester Form als Pulver, Schuppen oder Blatt in einer Zwei-Phasen Reaktion mit Vernetzern wie Formaldehyd, Glyoxal, Glutarsäuredialdehyd, Dicarbonsäurechloriden und/oder Diisocyanaten umgesetzt.

Der Vernetzer wirkt dabei auf die gequollene, nicht gelöste Gelatine ein. Dieses Verfahren und die daraus erhaltenen Produkte sind mit erheblichen Nachteilen behaftet, da die verwendeten Vernetzer erhebliche Zellschädigungen bewirken können.

Zudem ist das Verfahren nicht oder nur schwer reproduzierbar. Die Vernetzung hängt nicht nur von der Konzentration der verwendeten Vernetzer ab, sondern auch von Parametern wie Temperatur und Einwirkzeit der Reaktanden. Ferner sind die effektive Oberfläche und das mittlere Molekulargewicht der handelsüblichen Gelatinearten deutlichen Schwankungen unterworfen, so daß auch die Eigenschaften des vernetzten Hydrogels schwer voraussehbar sind.

Weiterhin ist in EP-A-0 097 846 ein Verbandmaterial aus Hydrogelen auf Polyvinylakoholbasis beschrieben. Als Vernetzer wird Formaldehyd eingesetzt, der, wie eingangs erwähnt, physiologisch bedenklich ist.

Auch Schäume aus Polyvinylalkohol oder Collagen sind bekannt und gebräuchlich. Da deren Matrixsubstanzen aber die voranstehend beschriebenen Nachteile haben, sind sie zur Wundversorgung allenfalls bedingt geeignet.

Es war daher die Aufgabe der Erfindung, Hydrogelschäume zu entwickeln, welche nicht die Nachteile des Standes der Technik haben und als Wundversorgungsmittel geeignet sind. Außerdem sollten die Schäume nach reproduzierbaren Verfahren wirtschaftlich herstellbar sein.

Überraschend wurde gefunden, daß Hydrogelschäume,

a) basierend auf Gelatine und Wasser,

b) enthaltend gegebenenfalls Polyvinylalkohol

c) enthaltend einen Vernetzer, gewählt aus der Gruppe

ca) der Verbindungen mindestens dreiwertiger Metalle oder Halbmetalle, z.B. Borsäure, Borate, Aluminiumsalze, Titanate, Alkyltitanate

und/oder der Gruppe der

cb) organischen und/oder anorganischen Säuren und/oder deren Salzen, z.B. Gallussäure, Gallate, Phosphorsäuren, Phosphate, Dihydroxybenzoesäure, Dihydroxybenzoate

d) sowie gegebenenfalls enthaltend organische Weichmacher, und/oder Hilfs- und/oder Zusatzstoffe

den Nachteilen des Standes der Technik abhelfen und die Eigenschaften von dessen Produkten deutlich überragen.

Obwohl an sich bekannt ist, daß Zusatz von Borsäure die Viskosität von Polyvinylalkoholen erhöht (z.B. Hoechst-Firmenschrift Mowiol, aus 1984), findet sich jedoch nirgends der Hinweis darauf, daß die erfindungsgemäßen Vernetzer überhaupt vernetzend wirken. Im Zusammenhang mit den erfindungsgemäßen Hydrogelschäumen werden diese Substanzen im folgenden dennoch die Bezeichnung "Vernetzer" tragen.

Weiterhin war nicht vorherzusehen, daß die erfindungsgemäßen Zusammensetzungen zu Schäumen führen. Wie anhand von Vergleichsbeispiel 1 gezeigt werden wird, erhält man mit Polyvinylalkohol und erfindungsgemäßem Vernetzer allein, ohne Zusatz von Gelatine, keine Schäume.

Bevorzugt enthalten die erfindungsgemäßen

Hydrogelschäume

0,01 - 20,0 Gew.-%
Vernetzer
0,1 - 30,0 Gew.- %
Gelatine
30,0 - 0,0 Gew.-%
Polyvinylalkohol
0,0 - 50,0 Gew.-%
organische Weichmacher
50,0 - 90,0 Gew.-%
Wasser
bis zu etwa 20 Gew.-%
Hilfs- und Zusatzstoffe

bezogen auf das Gesamtgewicht der Zusammensetzung.

Besonders bevorzugt enthalten die erfindungsgemäßen Hydrogelschäume

0,01 - 10,0 Gew.-%
Vernetzer
1,0 - 15,0 Gew.-%
Gelatine
15,0 - 1,0 Gew.-%
Polyvinylalkohol
5,0 - 30,0 Gew.-%
organische Weichmacher
50,0 - 80,0 Gew.-%
Wasser
bis zu etwa 25 Gew.-%
Hilfs- und Zusatzstoffe

bezogen auf das Gesamtgewicht der Zusammensetzung.

Ganz besonders bevorzugt enthalten die erfindungsgemäßen Hydrogelschäume

0,05 - 5,0 Gew.-%
Vernetzer
2,0 - 15,0 Gew.-%
Gelatine
15,0 - 2,0 Gew.-%
Polyvinylalkohol
10,0 - 30,0 Gew.-%
organische Weichmacher
50,0 - 80,0 Gew.-%
Wasser
bis zu etwa 20 Gew.-%
Hilfs- und Zusatzstoffe

bezogen auf das Gesamtgewicht der Zusammensetzung.

Als Abbauprodukt des Kollagens kann die Gelatine je nach dem Grad des Abbaus recht unterschiedliche Molekulargewichte aufweisen. In Abhängigkeit davon besitzen die verschiedenen Gelatinesorten unterschiedliche Gallertfestigkeiten, die üblicherweise in sogenannten "Bloomgraden" angegeben werden.

Die Gallertfestigkeit wird ermittelt, indem eine Gelatineplatte, die 6 2/3 Gew.-% luftgetrocknete (also noch feuchte) Gelatine enthält und 10 Stunden bei 18 °C gestanden hat, mit einem Stempel von 1/2 Zoll Durchmesser belastet wird. Dabei dringt der Stempel in die Gelatine ein. Die Gelatine hat die Gallertfestigkeit von 1 Bloomgrad, wenn bei einer Eindringtiefe von 4 mm ein Gewicht von 1 Gramm auf dem Stempel lastet.

Zur Herstellung der erfindungsgemäßen Hydrogelschäume sind alle handelsüblichen Gelatinesorten mit Gallertfestigkeiten von 30 - 300 Bloomgraden geeignet.

Polyvinylalkohole sind im Handel erhältlich, beispielsweise unter den Handelsbezeichnungen Polyviol® oder Mowiol®. Es handelt sich dabei um polymere Verbindungen der allgemeinen Formel

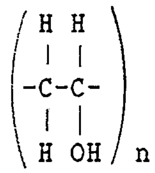

Dabei kann n erfindungsgemäß so gewählt werden, daß der Polyvinylalkohol im Molekulargewichtsbereich von 10 000 bis 250 000 liegt.

Bevorzugte Vernetzer sind insbesondere Borax und Kaliumaluminiumsulfat (Kaliumalaun), aber auch die verschiedenen Borsäuren sowie andere Alaune, beispielsweise Eisenalaun, sind erfindungsgemäß hervorragend geeignet. Von den Phosphorsäuren ist die ortho-Phosphorsäure bevorzugt.

Weitere günstige Vernetzer sind Magnesiumsulfat, Aluminiumsulfat, Dihydroxybenzoesäure und deren Salze, Gallussäure und Gallate, Vanadin (III) - chlorid, Aluminiumhydroxid-acetat.

Auch die anderen genannten Vernetzer führen zu äußerst vorteilhaften Ausführungsformen der vorliegenden Erfindung. Von den Alkyltitanaten hat sich das Tetraethylorthotitanat als bevorzugt herausgestellt.

Die vernetzten Hydrogele können bis zu 50%, bezogen auf die Trockensubstanz, an Weichmachern, vorzugsweise flüssigen Polyolen enthalten, wobei das bevorzugte Polyol mit der geringsten Zelltoxizität das Glycerin ist. Andere Polyole, die an sich eine gewisse Zelltoxizität aufweisen, können dennoch vorteilhaft eingesetzt werden, wenn sie besser verträglich mit den vernetzten Hydrogelen sind, zum Beispiel:
Ethylenglycol, flüssige Polyethylenglycole verschiedener Molmasse, Propylenglycol-1,2, flüssige Polypropylenglycole-1,2 verschiedener Molmasse, Propylenglycol-1,3, flüssige Polypropylenglycole-1,3 verschiedener Molmasse, Zitronensäuretriethyl- oder trimethylester, Milchsäuremethyl- oder -ethylester, Glycolsäuremethyl- oder -ethylester oder 2-Ethyl-2 (hydroxymethyl)-1,3-propandiol.

Vorteilhafte Weichmacher sind insbesondere

auch Mono-, Di-, oder Oligosaccharide, vorzugsweise Rohrzucker oder Sorbit.

Diese Verbindungen schützen die erfindungsgemäßen Hydrogele vor dem Austrocknen. Dies ist insbesondere dann günstig, wenn das fertige Hydrogel nicht wasserdicht oder wasserdampfundurchlässig verpackt wird.

Es sei darauf hingewiesen, daß die Handelsprodukte, insbesondere die Gelatine und der Polyvinylalkohol, aber auch die Vernetzer und die Weichmacher verarbeitungsbedingt einen gewissen Wassergehalt aufweisen. Wenn nicht anders angegeben, bedeuten die Angaben für die einzelnen Stoffe trockene Reinsubstanz.

Die Herstellung der erfindungsgemäßen Hydrogelschäume erfolgt derart, daß zunächst die Hydrogelbasis, d.i. Gelatine, oder ein Gemisch aus Gelatine und Polyvinylalkohol, mit Wasser und, falls gewünscht, dem Weichmacher, verrührt wird. Dabei ist es vorteilhaft, das Gemisch zu erwärmen, vorzugsweise auf Temperaturen von 60 - 100° C, insbesondere auf Temperaturen von 70 - 95° C. Der Lösung, die man auf diese Weise erhält, wird der Vernetzer zugefügt. Durch das Reaktionsgemisch, welches kräftig gerührt werden sollte, wird ein Gasstrom geleitet. Der sich nach einiger Zeit bildende Schaum kann weiter verarbeitet werden, beispielsweise zu flächigen Gebilden ausgestrichen werden.

Bevorzugt ist ein Verfahren zur Herstellung von Hydrogelschäumen, in welchem

* Gelatine, oder ein Gemisch aus Gelatine und Polyvinylalkohol, mit Wasser und, falls gewünscht, dem Weichmacher, verrührt wird,
* dem Gemisch, das man auf diese Weise erhält, der Vernetzer im Sinne der Erfindung zugefügt wird,
* das so entstandene Reaktionsgemisch kräftig gerührt wird,
* durch das Reaktionsgemisch ein Gasstrom geleitet wird,
* der so entstandene Schaum gegebenenfalls zu flächigen Gebilden ausgestrichen wird oder gegebenenfalls auf einen flächigen Träger aufgetragen wird.

Besonders vorteilhaft können die erfindungsgemäßen Hydrogelschäume nach an sich bekannten Verfahren auf flächige Träger, z.B. Gewebe, Gewirke, Vliese, Folien oder dergleichen aufgetragen werden. Die so erhaltenen beschichteten Träger können zudem mit einer Selbstklebemasse versehen werden.

Es ist auch möglich und vorteilhaft, Gegenstände aus den erfindungsgemäßen Hydrogelschäumen anzufertigen, welche nicht flächig, sondern ausgeprägt raumfüllend sind.

Der fertige Schaum, vorzugsweise die vorabbeschriebenen flächigen Gebilde, vorteilhaft aber auch die mit Hydrogelschaum beschichteten Träger, können beispielsweise dazu dienen, Oberflächenwunden oder chirurgische Wunden zu versorgen.

Die nachfolgenden Beispiele sollen die Erfindung erläutern, ohne daß aber beabsichtigt ist, die Erfindung auf diese Beispiele zu beschränken.

Beispiel 1

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 73 Gewichtsteilen Wasser und 16 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 0,1 Gewichtsteilen Borax versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 2

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 64,5 Gewichtsteilen Wasser und 23 Gewichtsteilen Sorbit (enthaltend ca. 30 Gew.-% Wasser)
wird 2 Stunden unter Rühren auf 85 - 90° C erhitzt. Es bildet sich eine Lösung, die dann mit 1,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 3

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 68 Gewichtsteilen Wasser, 11,5 Gewichtsteilen Sorbit (enthaltend ca 30 Gew.-% Wasser) und 16 Gewichtsteilen Glycerin (enthaltend ca. 15 Gew.-% Wasser)

wird 2 Stunden unter Rühren auf 85 - 90° C erhitzt. Es bildet sich eine Lösung, die dann mit 1,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 4

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 74,2 Gewichtsteilen Wasser und 14 Gewichtsteilen Saccharose
wird 2 Stunden unter Rühren auf 85 - 90° C erhitzt. Es bildet sich eine Lösung, die dann mit 0,7 Gewichtsteilen Kalium-Aluminiumsulfat und 0,1 Gewichtsteilen Borax versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 5

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 72,2 Gewichtsteilen Wasser und 16 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 0,8 Gewichtsteilen Tetraethyltitanat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 6

Eine Mischung aus
8 Gewichtsteilen Gelatine (enthaltend ca. 10 Gew.-% Wasser), 7 Gewichtsteilen Polyvinylalkohol (Molekulargewicht ca. 75 000), 72,9 Gewichtsteilen Wasser und 12 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 0,1 Gewichtsteilen Borax versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 7

Eine Mischung aus
2 Gewichtsteilen Gelatine (enthaltend ca. 10 Gew.-% Wasser), 8 Gewichtsteilen Polyvinylalkohol (Molekulargewicht ca. 75 000), 70,5 Gewichtsteilen Wasser und 16 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 3,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 8

Eine Mischung aus
11 Gewichtsteilen Gelatine (enthaltend ca. 10 Gew.-% Wasser), 72,9 Gewichtsteilen Wasser und 16 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 0,1 Gewichtsteilen Borax versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 9

Eine Mischung aus

4 Gewichtsteilen Gelatine (enthaltend ca. 10 Gew.-% Wasser), 7 Gewichtsteilen Polyvinylalkohol (Molekulargewicht ca. 75 000), 72,4 Gewichtsteilen Wasser und 16 Gewichtsteilen Glycerin (enthaltend ca. 15 Gew.-% Wasser)
wird 2 Stunden unter Rühren auf 85 - 90° C erhitzt. Es bildet sich eine Lösung, die dann mit 0,6 Gewichtsteilen Gallussäure versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 10

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 71,5 Gewichtsteilen Wasser, 14 Gewichtsteilen Glycerin (enthaltend ca. 15 Gew.-% Wasser) und 2 Gew.-% Panthenol
wird 2 Stunden unter Rühren auf 85 - 90° C erhitzt. Es bildet sich eine Lösung, die dann mit 1,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Beispiel 11

Eine Mischung aus
4 Gewichtsteilen Gelatine (enthaltend ca. 10 Gew.-% Wasser), 7 Gewichtsteilen Polyvinylalkohol (Molekulargewicht ca. 75 000), 71,5 Gewichtsteilen Wasser, 13 Gewichtsteilen Glycerin (enthaltend ca. 15 Gew.-% Wasser), 1,5 Gew.-% Panthenol und 1,5 Gew.-% Ascorbylpalmitat
wird 2 Stunden unter Rühren auf 85 - 90° C erhitzt. Es bildet sich eine Lösung, die dann mit 1,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird ein streichfähiger Schaum erhalten, der zu Blättern von ca. 1 mm Stärke ausgestrichen werden kann.

Vergleichsbeispiel 1

Eine Mischung aus
11 Gewichtsteilen Polyvinylalkohol (Molekulargewicht ca. 75 000), 64,5 Gewichtsteilen Wasser und 23 Gewichtsteilen Sorbit (enthaltend ca. 30 Gew.-% Wasser)
wird 2 Stunden unter Rühren auf 85 - 90° C erhitzt. Es bildet sich eine Lösung, die dann mit 1,5 Gewichtsteilen Kalium-Aluminiumsulfat versetzt wird. Nach einer weiteren Stunde wird die Badtemperatur auf 60° C gesenkt. Die Rührgeschwindigkeit wird auf 1 000 Umdrehungen/Minute eingestellt. Dabei wird über einen Zeitraum von 2 Stunden ein kontinuierlicher Gasstrom durch die Mischung geleitet.

Es wird kein Schaum erhalten.

**Patentansprüche**

**1.** Hydrogelschäume,
a) basierend auf Gelatine und Wasser,
b) enthaltend gegebenenfalls Polyvinylalkohol
c) enthaltend einen Vernetzer, gewählt aus der Gruppe
ca) der Verbindungen mindestens dreiwertiger Metalle oder Halbmetalle, z.B. Borsäure, Borate, Aluminiumsalze, Titanate, Alkyltitanate
und/oder der Gruppe der
cb) organischen und/oder anorganischen Säuren und/oder deren Salzen, z.B. Gallussäure, Gallate, Phosphorsäuren, Phosphate, Dihydroxybenzoesäure, Dihydroxybenzoate
d) sowie gegebenenfalls enthaltend organische Weichmacher, und/oder Hilfs- und/oder Zusatzstoffe

**2.** Hydrogelschäume nach Anspruch 1, dadurch gekennzeichnet, daß sie
0,01 - 20,0 Gew.-%
Vernetzer im Sinne der Erfindung
0,1 - 30,0 Gew.-%
Gelatine
30,0 - 0,0 Gew.-%
Polyvinylalkohol
0,0 - 50,0 Gew.-%
organische Weichmacher
50,0 - 90,0 Gew.-%
Wasser
bis zu etwa 20 Gew.-%
Hilfs- und Zusatzstoffe
bezogen auf das Gesamtgewicht der Zusammensetzung, enthalten.

**3.** Hydrogelschäume nach Anspruch 1 oder 2, dadurch gekennzeichnet, daß sie
0,01 - 10,0 Gew.-%
Vernetzer im Sinne der Erfindung
1,0 - 15,0 Gew.-%
Gelatine
15,0 - 1,0 Gew.-%
Polyvinylalkohol
5,0 - 30,0 Gew.-%
organische Weichmacher
50,0 - 80,0 Gew.-%
Wasser
bis zu etwa 25 Gew.-%
Hilfs- und Zusatzstoffe
bezogen auf das Gesamtgewicht der Zusammensetzung, enthalten.

**4.** Hydrogelschäume nach einem der Ansprüche 1 - 3, dadurch gekennzeichnet, daß sie
0,05 - 5,0 Gew.-%
Vernetzer im Sinne der Erfindung
2,0 - 15,0 Gew.-%
Gelatine
15,0 - 2,0 Gew.-%
Polyvinylalkohol
10,0 - 30,0 Gew.-%
organische Weichmacher
50,0 - 80,0 Gew.-%
Wasser
bis zu etwa 20 Gew.-%
Hilfs- und Zusatzstoffe
bezogen auf das Gesamtgewicht der Zusammensetzung, enthalten.

**5.** Hydrogelschäume nach einem der Ansprüche 1 - 4, dadurch gekennzeichnet, daß die Vernetzer gewählt werden aus der Gruppe Borax, Borsäure, Tetraethylorthotitanat, ortho-Phosphorsäure und Kalium-Aluminiumsulfat.

**6.** Verfahren zur Herstellung von Hydrogelschäumen nach einem der Ansprüche 1 - 5, dadurch gekennzeichnet, daß
e) Gelatine, oder ein Gemisch aus Gelatine und Polyvinylalkohol, mit Wasser und, falls gewünscht, dem Weichmacher, verrührt wird,
f) dem Gemisch, das man auf diese Weise erhält, der Vernetzer im Sinne der Erfindung zugefügt wird,
g) das so entstandene Reaktionsgemisch kräftig gerührt wird,
h) durch das Reaktionsgemisch ein Gasstrom geleitet wird,
i) der so entstandene Schaum gegebenenfalls zu flächigen Gebilden ausgestrichen wird oder gegebenenfalls auf einem flächigen Träger aufgetragen wird.

**7.** Flächige Gebilde aus Hydrogelschaum nach einem der Ansprüche 1 - 5, erhältlich aus einem Verfahren nach Anspruch 6.

**8.** Flächige Träger, beschichtet mit Hydrogelschaum nach einem der Ansprüche 1 - 6.

**9.** Verwendung von Hydrogelschäumen nach einem der Ansprüche 1 - 5 , 7 und 8 zum Schutze von Wunden vor der Austrocknung, zum Aufsaugen von Wundsekret, als Matrix für Wirkstoffe aller Art, als Basis für die Besiedelung mit autologen oder heterologen Hautzellen.